# EUROPEAN PATENT SPECIFICATION

(11) **EP 0 372 685 B1**
(45) Date of publication and mention of the grant of the patent: **14.06.1995**
(21) Application number: 89309183.5
(22) Date of filing: 11.09.1989
(51) Int. Cl.: C07C 37/72, C07C 37/08

(54) **Process for recovering phenol**
Verfahren zur Wiedergewinnung des Phenols
Procédé de récupération de phénol

(30) Priority: 02.12.1988 IT 2282388
(43) Date of publication of application: 13.06.1990
(73) Proprietor: ENICHEM S.p.A., 20124 Milano (IT)
(72) Inventor: Penzo, Renzo, I-46100 Mantova (IT); Reggiani, Paolo, I-46030 Virgilio Mantova (IT); Spaggiari, Claudio, I-46100 Mantova (IT)
(74) Representative: Jones, Helen Marjorie Meredith

(56) References cited:
- DE-B- 1 094 758
- DE-B- 1 101 435
- GB-A- 674 754
- US-A- 2 200 370

## Description

The present invention relates to a process for recovering phenol from a rectification purge which contains organic impurities.

When phenol is manufactured cleavage of cumene hydroperoxide, a crude phenol is obtained, which has to be purified by hydrodistillation or by extractive distillation; in both processes, two distillation columns are used. In the first column, at the top as the overhead, one separates by-products, mostly a light cut, containing mesityl oxide, hydroxyacetone, cumene, alpha-methylstyrene, 2-methyl-benzofuran and other organic substances, which are present (as impurities) in the crude phenol; in the second column, the pure phenol is distilled. The purge (overhead) obtained from the top of the first column contains phenol, in a concentration from 20% to 90% by weight, usually from 50% to 80% by weight. The phenol contained in said purge can be recovered, as it is known, by salification with a strong base, for instance caustic soda; the resulting sodium phenate remains dissolved in the aqueous phase, which is then acidified for example with H₂SO₄, H₂CO₃ or another acid, thereby obtaining again free phenol. To obtain a complete (or almost complete) displacement of phenol to the aqueous phase, in the form of salt, it is necessary to operate with a base excess and, should NaOH be used as a base, the excess usually amounts to a quantity ranging from 1% to 30% of the maximum amount convertible to sodium phenate; the base consumption is therefore a quite considerable burden.

EP-A-0,028,522 suggests, as an alternative, to recover phenol from the purge by means of steam distillation, but also in such a process the costs connected with the use of steam is very significant.

In principle, it would be possible to recover phenol by means of an extraction with H₂O: this experiment would allow to avoid any waste of NaOH or steam. The distribution coefficient of phenol between aqueous phase and organic purge would however require to feed a considerable water amount to the plant, which would result in an increase of the power (and reactants) consumption and the cost in the whole would be even higher than the cost arising from the recovery by means of salification with NaOH.

DE-A-1094758 and DE-A-1101435 disclose the use of alkali metal phenolate for extracting phenol from a phenol-containing oil or tar fraction. The concentration of alkali metal phenolate used in the aqueous extraction liquid is at least 25%.

GB-A-674754 discloses the use of aqueous alkali metal phenolate solutions having a concentration of at least 20% by weight for extracting phenol from mixtures of hydrocarbons and phenols.

The applicant has now found a method, which allows to recover phenol from said rectification purge in a very simple manner and with yields like the ones obtainable from the salififcation with NaOH or from the steam distillation, while avoiding any consumption of NaOH or of steam.

In its widest aspect, the invention comprises a process for recovering phenol from a rectification purge, containing from 20 to 90% by weight of phenol, besides various foreign matters, in particular mesityl oxide, hydroxy-acetone, alpha-methylstyrene and 2-methylbenzofuran, which process consists of subjecting said purge to an extraction operation, using, as an extracting solvent, an aqueous solution of an alkali metal phenate, for instance sodium phenate (C₆H₅ONa), characterised in that the aqueous solution contains from 1 to 15% by weight of an alkali metal phenate which has been obtained in a process for the synthesis of phenol by cleavage of cumene hydroperoxide.

In a process for the synthesis of phenol, by cleavage of cumene hydroperoxide, one can use various aqueous solutions of sodium phenate, which may be available in the plant. The total amount of phenate solutions available is usually by far greater than the purge mass to be treated. The applicant has surprisingly found that extraction by the sodium phenate solution can be as effective as the conventional extraction with soda. Indeed it is possible to carry out said phenol recovery practically without adding to the cost. In other words, the advantage of the invention resides in that the presence of an alkali metal phenate causes an unexpected increase in the distribution coefficient of phenol between aqueous phase and organic phase, thereby promoting the phenol solubilization in the aqueous phase.

The aqueous solution of sodium phenate, which is utilized in this process may contain, besides said phenate, also small amounts of free NaOH. In such a process, a salification of phenol will take place to a small extent; however that does not of course, defract from the usefulness of the invention.

The invention can be carried out in various ways; the extraction process, for instance, can be performed to particular advantage in conjunction with any or all of the following features:
a) the aqueous solvent contains from 1 to 15% and preferably from 5 to 15% by weight of sodium phenate;
b) the extraction temperature ranges from room temperature to 90°C and preferably from 40°C to 80°C;
c) the extracting solvent amount ranges from 1 to 15 and preferably from 5 to 15 parts by weight per each part of purge;
d) the extraction is performed continuously, the reactants being fed (counter-current) into an apparatus consisting of two (or more than two) mixers and of two (or more than two) decanters, or into an apparatus, which allows a plurality of extraction steps, without any need to separate the two flows, preferably taking care that the organic phase be the continuous phase and that the aqueous phase be the dispersed phase;
e) the apparatus e.g. as in d) can advantageously have a tubular shape and can be filled with packing bodies, preferably of stainless steel or of another material preferentially wetted by the organic phase;
f) the packing bodies in e) are RASCHIG rings or PALL rings.

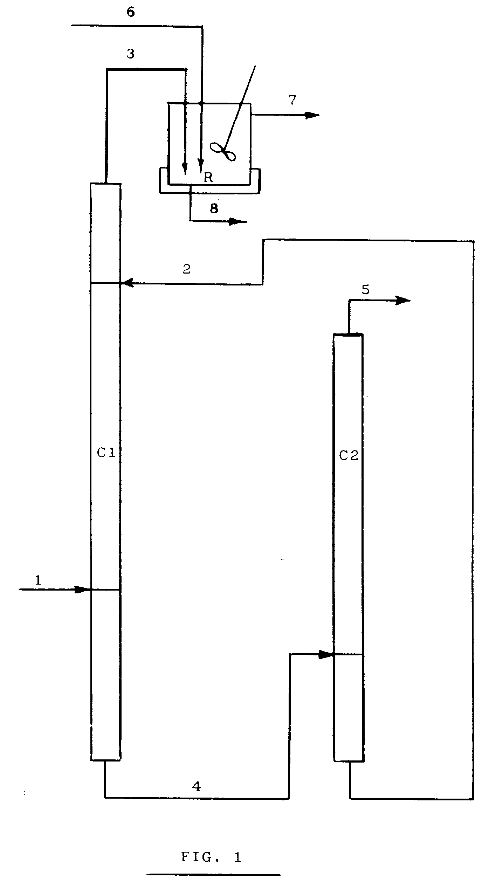

Figures 1 to 3 each illustrate diagrammatic representatives of three embodiments of the process of the invention.

The following examples illustrate some preferred embodiments of the invention.

### Example 1

### Part "A" (extractive distillation)

Following figure 1, rough phenol (1), having a titre of about 95% (by weight) and coming from the cleavage of cumene hydroperoxide, (already separated from acetone, from most of cumene and from high-boiling impurities), was fed to extraction column C1, which was fed also with a mixture of diethylene glycol (DEG) and phenol (2), coming from the bottom of column C2. The separation and removal from the column of almost all the impurities (3) (in particular alpha- methylstyrene and other hydrocarbons, hydroxyacetone, mesityl oxide, 2-methyl-benzofuran and so on) occurred at the top of column C1, whilst a mixture (4) of phenol and DEG was obtained from the bottom of column C1 and fed to column C2. From the top of column C2, pure phenol (5) was obtained, whilst from the bottom a phenol/DEG mixture was obtained, which was recycled to column C1. The refluxes (not indicated in the figure) of both columns were realized with their respective head condensates.

### Part "B" (recovery of phenol from the purge)

100g of overhead product (3) of column C1, containing 60% by weight of phenol, 1% by weight of water and 39% by weight of organic impurities, were fed to an extractor (R) having a volume of about 1,000 ml, equipped with a heating jacket, baffles and a rotary stirrer (500 r.p.m.). Said impurities consisted (for the most part) of mesityl oxide, hydroxyacetone, alpha-methylstyrene and 2-methyl-benzofuran. 500g of an aqueous solution (6) (extractant), containing 15% by weight of sodium phenate, flowing from another area of the phenol synthesis plant, were also fed to the extractor R. The temperature of the mixture was raised to 50°C, stirring was carried on for 15 minutes and the whole was then allowed to settle (still at 50°C) for a further period of 60 minutes. An organic (upper) phase (7) and an aqueous (lower) phase (8) were recovered. The results, reported in table 1, reveal a remarkable phenol shifting towards the aqueous phase.

### Example 2

Example 1 was repeated using, as an extractant, an aqueous solution containing 10% by weight of sodium phenate. The data and results are recorded in table 1.

### Example 3

Example 1 was repeated using, as an extractant, an aqueous solution containing 5% by weight of sodium phenate. The data and results are recorded in table 1.

### Example 4 (comparative)

Example 1 was repeated replacing the addition of sodium phenate by the introduction of 500g of pure (deionized) water. As seen from table 1, the phenol shifting to the aqueous phase is much lower, when compared with the values obtainable when sodium phenate is present in the extractant, in examples 1 to 3.

### Examples 5-6

Example 3 was repeated, varying the extraction temperature (70° and 90°C respectively). The results are recorded in table 2. They prove that even a slight raising of the temperature leads to a considerable increase in the extraction yield.

### Example 7

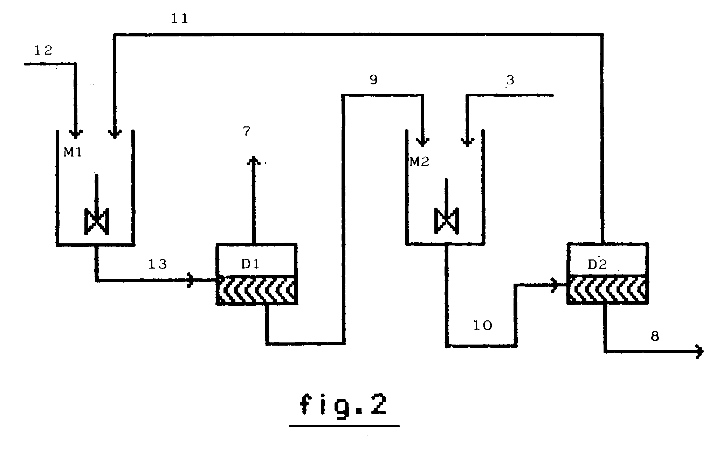

Example 3 was repeated carrying out the extraction by means of the apparatus lay-out illustrated in figure 2, said system comprising two mixers (M1 and M2) and two decanters (D1 and D2). According to figure 2, 100 g/h of the overhead product (3) of column C1, having the composition described in example 1, were continuously fed to mixer M2 along with the aqueous phase (9), separated in decanter D1. The effluent from M2 (10) was conveyed to decanter D2, where the aqueous phase (8), containing all the extracted phenol, and the organic phase (11) were separated, the latter being fed to mixer M1 along with 500 g/h of aqueous solution (12), comprising 5% by weight of sodium phenate. The liquid (13), flowing out from M1 was transferred to separator D1, where the aqueous phase (9), which was transferred to mixer M2, and the organic phase (7) were separated. Data and results are recorded in table 2, from which it is clear that, at the end of the extraction, only 9.7 g of phenol remained dissolved in 39.5 g of organic phase, what means that 83.8% of the starting phenol had been recovered in the aqueous phase.

### Example 8

A tubular extraction apparatus, equipped with a heating jacket, having an inner diameter of 75 mm and a height of 9 m (divided into three sections of 3 m each), was loaded with ceramic BERL saddles; size: 3/8" (9.5mm). To the extractor bottom there were continuously fed 5 l/h of the rectification purge of example 3, in countercurrent with 50 l/h (ratio = 10:1) of an extracting aqueous solution containing 5% by weight of sodium phenate; the temperature was kept at 50°C by means of warm water circulation. The aqueous flow formed the continuous phase while the organic flow formed the dispersed phase. Data and results are recorded in table 3 from which it is clear that only 5.1% by weight of phenol was still in the organic phase, at the end of the extraction, and that 96.4% of the starting phenol had been recovered in the aqueous phase.

### Example 9

Example 8 was repeated, replacing the ceramic saddles by stainless steel (AISI 316) RASCHIG rings; size: 3/8" (9.5mm). The organic phase formed the continuous phase whereas the aqueous phase, contrary to example 8, formed the dispersed phase. Data and results are recorded on table 3, from which it is clear that only 0.20% by weight of phenol was remaining, at the end of the extraction, in the organic phase, and that 99.9% of the starting phenol had been recovered in the aqueous phase. Said residual phenol percentage in the impurities (0.20%) was lower than the phenol amount (0.30%), which remain after a conventional treatment was carried out. The conventional treatment was carried out in a reactor equipped with a rotary stirrer, with a solution at 30% by weight of NaOH in an amount equal to 0.6% by weight of the aqueous phase. Surprisingly, the excellent result was obtained in the process of the invention without any consumption of caustic soda.

### Example 10

### Part "A" (hydrodistillation)

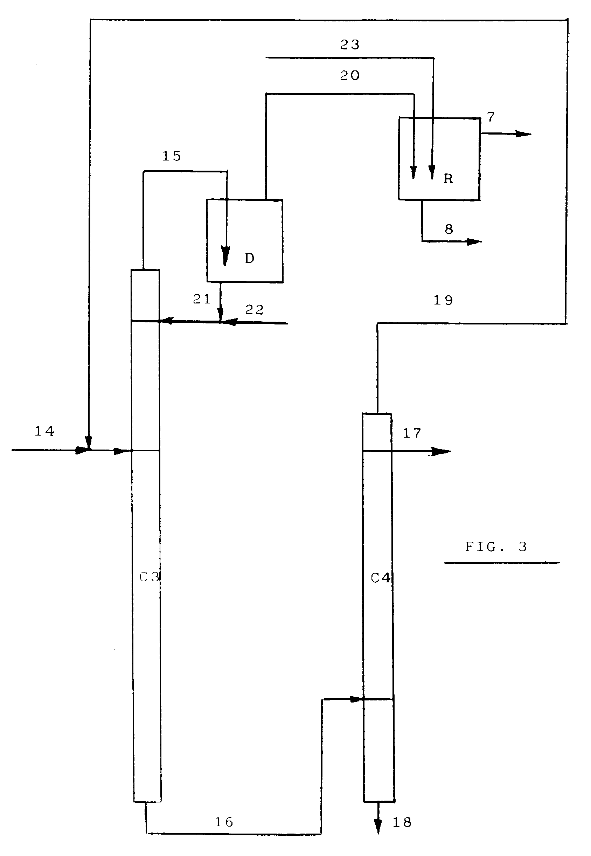

According to figure 3, crude phenol (14) was fed to column C3. The separation and removal from the column of the impurities (15) occurred at the top of column C3, whilst, from the bottom, a phenol stream (16) was obtained, which contained a very low amount of high-boiling foreign matters (and of H₂O) and which was fed to a second column C4; from a side cut, of this latter column, pure phenol (17) was obtained, whilst from its bottom a mixture of phenol and high-boiling products (18) was obtained, which was recycled to the process operations occurring upstream of column C3. From the top of C4, a small purge of phenol and water (19) was obtained, which was fed again to column C3. The overhead products (15) of the first column C3 were made to flow to reflux tank D, from which the impurities were removed, as organic phase (20), whilst the aqueous phase (21) was recycled as a reflux to the top of column C3, along with fresh make-up water (22), balancing the loss in the organic phase (20). Said organic phase (20) came then into contact with a sodium phenate solution (23), as it is described in detail in part "B" below.

### Part "B" (phenol recovery from the purge)

100g of organic phase (20), coming from separator (D) and containing 60% by weight of phenol, 10% by weight of water and 30% by weight of organic impurities, were fed to an extraction apparatus similar to apparatus R of example 1. The impurities consisted, for the most part, of mesityl oxide, hydroxyacetone, alpha- methylstyrene and 2-methyl-benzofuran. By working up as in example 1, the results recorded in table 4 were obtained, which reveal a remarked shifting of phenol towards the aqueous phase.

### Example 11

Example 10 was repeated using, as an extractant, an aqueous solution containing 10% by weight of sodium phenate. The data and results are recorded in Table 4.

### Example 12

Example 10 was repeated using, as an extractant, an aqueous solution containing 5% by weight of sodium phenate. The data and results are recorded in Table 4.

### Examples 13-14

Example 12 was repeated, varying the extraction temperature (70° and 90°C, respectively). The results are recorded in table 5.

### Example 15

Example 12 was repeated, carrying out the extraction by means of the apparatus illustrated in figure 2, consisting of two mixers (M1 and M2) and of two decanters (D1 and D2), as it is described in example 7. Data and results are recorded in table 5, from which it is clear that, at the end of the extraction, only 0.8g of phenol remained in 31g of the organic phase, which means that 98.7% of the starting phenol had been recovered in the aqueous phase.

### Example 16

To the bottom of the tubular extractor of example 8, 5 litres/h of the hydrodistillation purge of example 12 were continuously fed (in countercurrent) with 25 l/h (ratio by volume = 5:1) of an extracting aqueous solution containing 5% by weight of sodium phenate; the temperature was kept at 50°C by hot water circulation. The aqueous phase was the continuous phase and the organic phase was the dispersed phase. Data and results are recorded in table 6, from which it is clear that at the end of the extraction, only 0.32% by weight of phenol remained in the organic phase, and that 99.8% of the starting phenol had been recovered in the aqueous phase.

### Example 17

Example 16 was repeated, replacing the saddles by stainless steel (AISI 316) RASCHIG rings (3/8"). The organic phase was the continuous phase, whereas the aqueous phase, contrary to example 16, was the dispersed phase. The data and results are indicated in table 6, from which it is clear that, at the end of the reaction, less than 0.05% by weight of phenol remained in the organic phase and that more than 99.97% of the starting phenol had been recovered in the aqueous phase. The residual phenol (below 0.05% in the impurities) was by far lower than the phenol amount (0.25%) which remains after a treatment carried out according to a conventional technique, ie in an extractor equipped with a rotary stirrer, using a solution at 30% by weight of NaOH, in such amount as to have, after salification, a NaOH excess equal to 0.6% by weight of the aqueous phase. This excellent and surprising result was obtained without any consumption of caustic soda.

### Examples 18 and 19

Examples 9 and 17 were repeated, replacing the RASCHIG rings by PALL rings; analogous results were obtained.

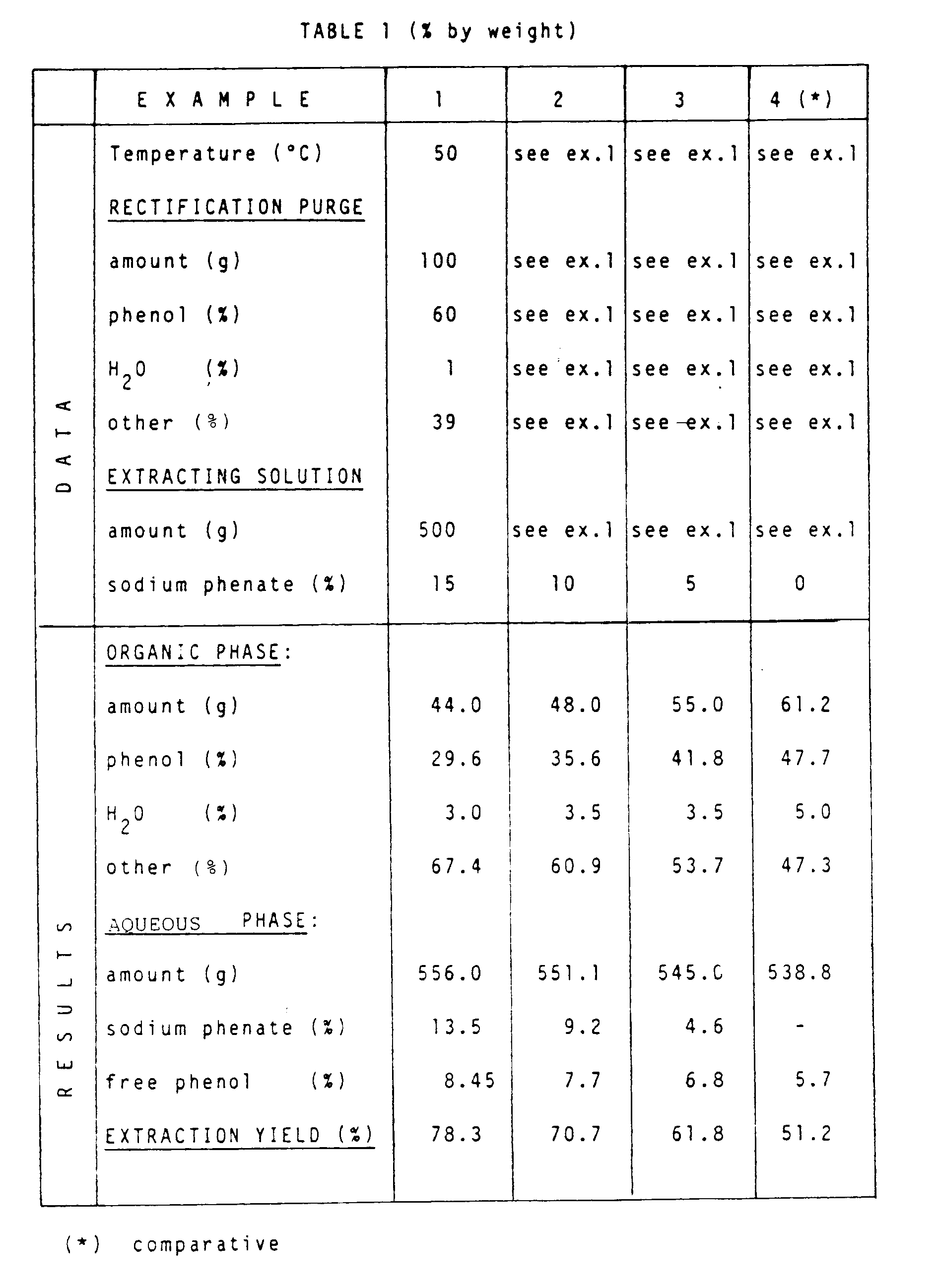

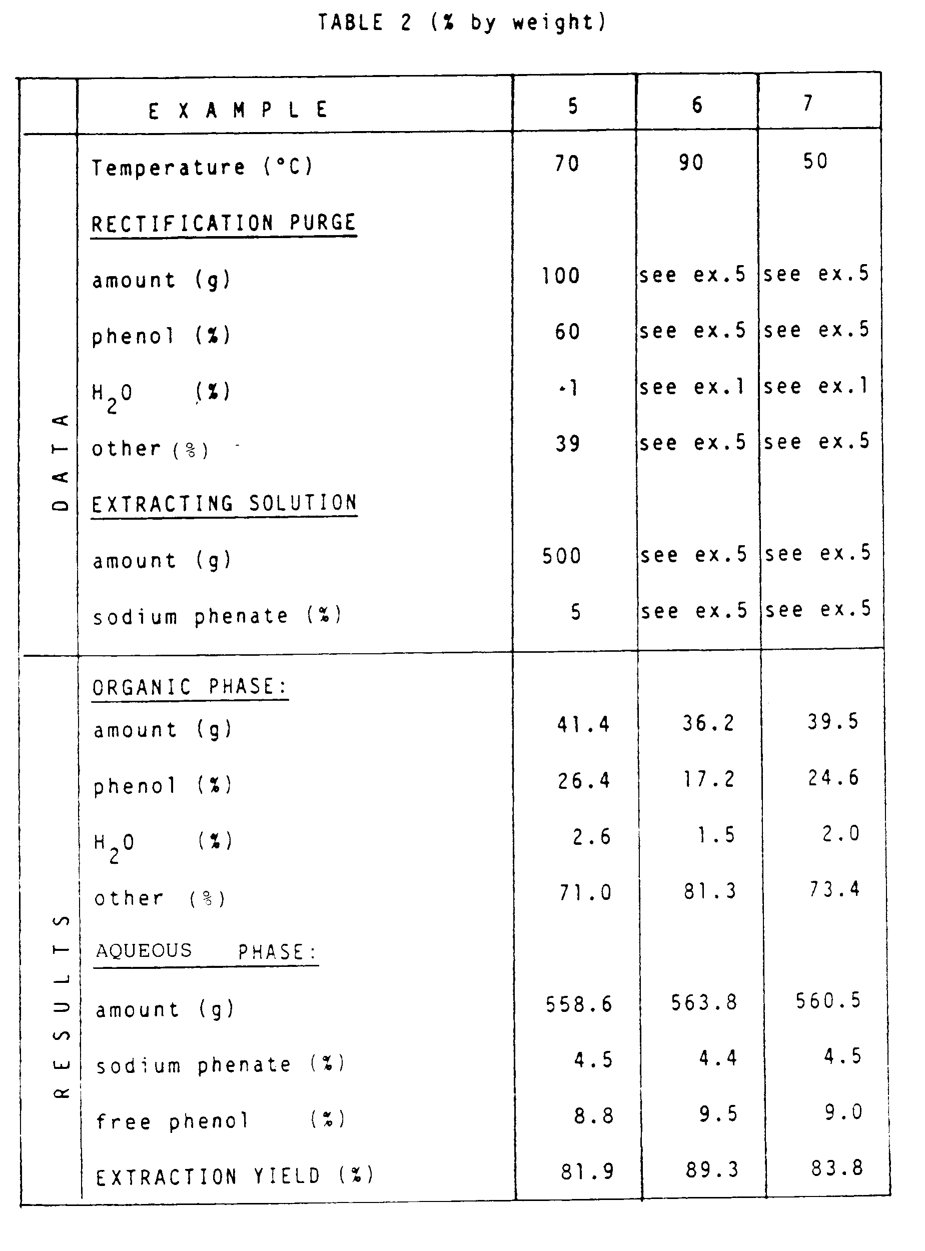

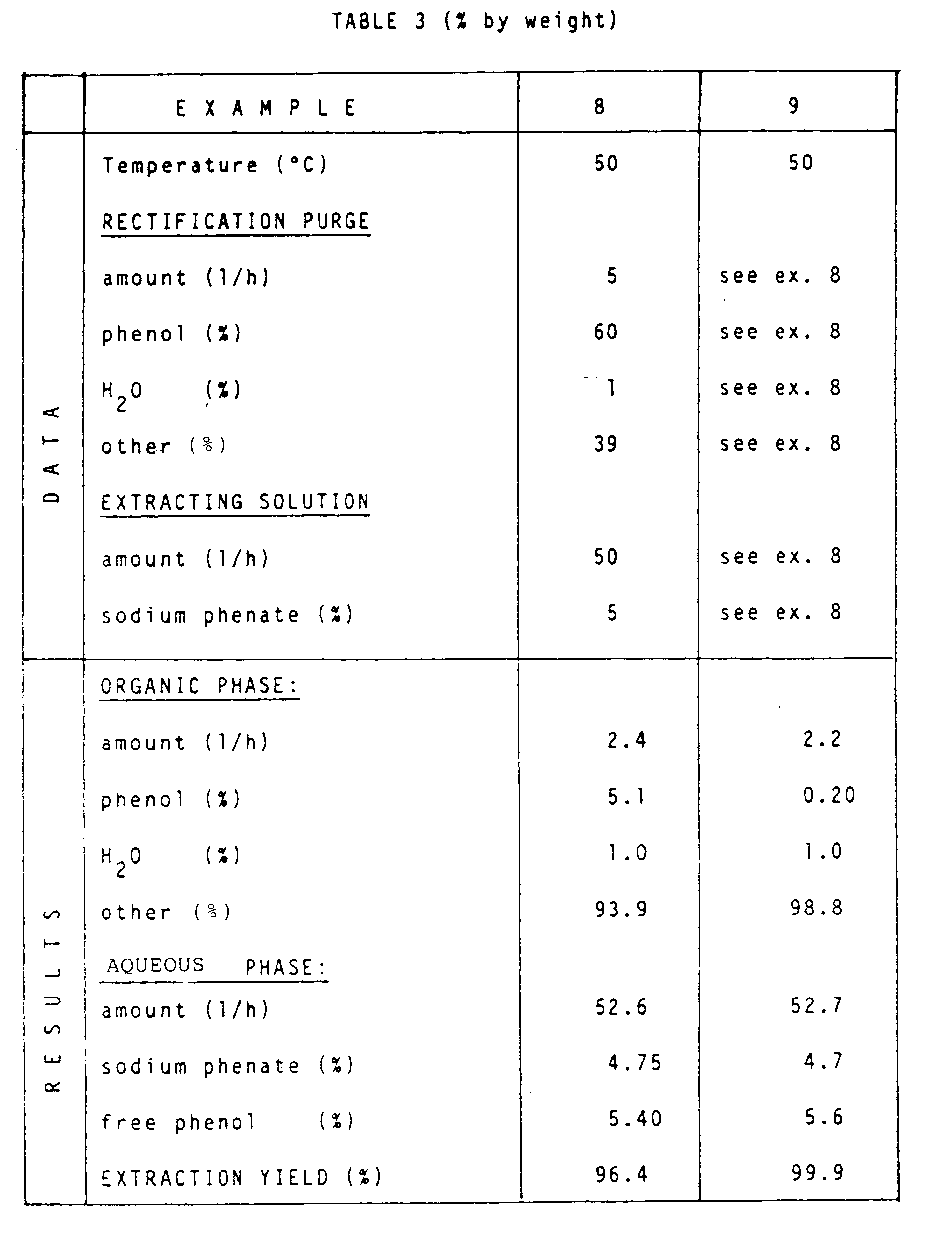

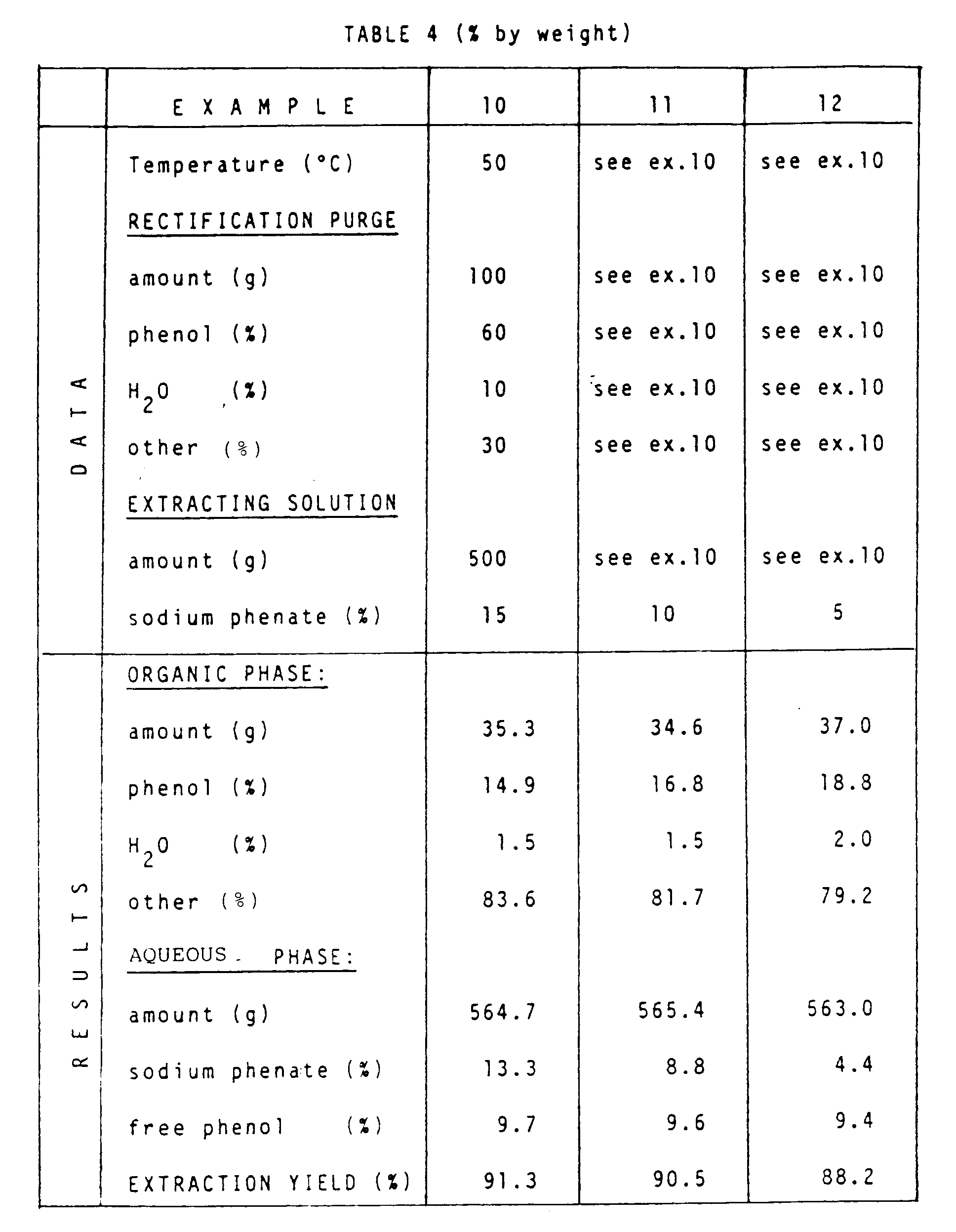

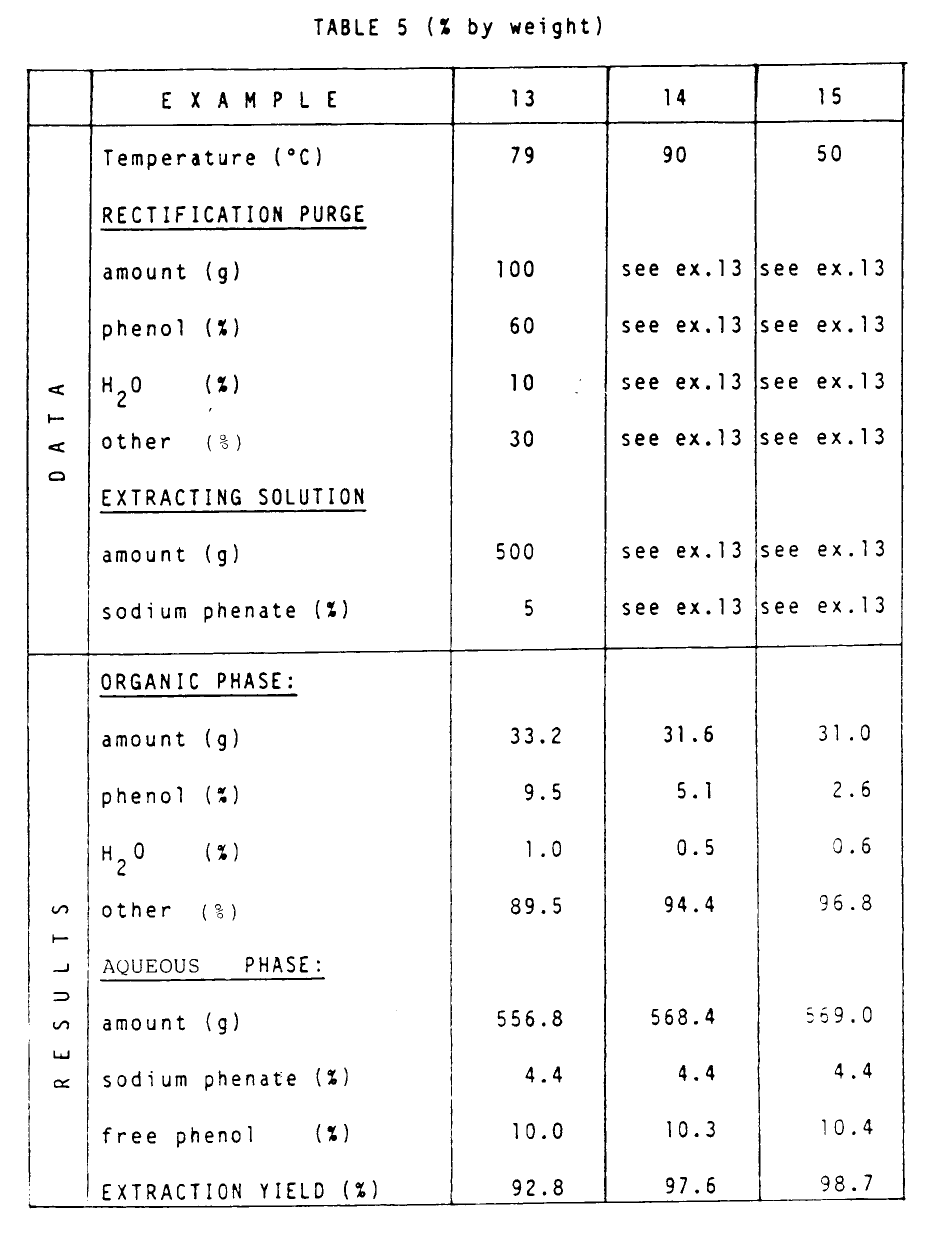

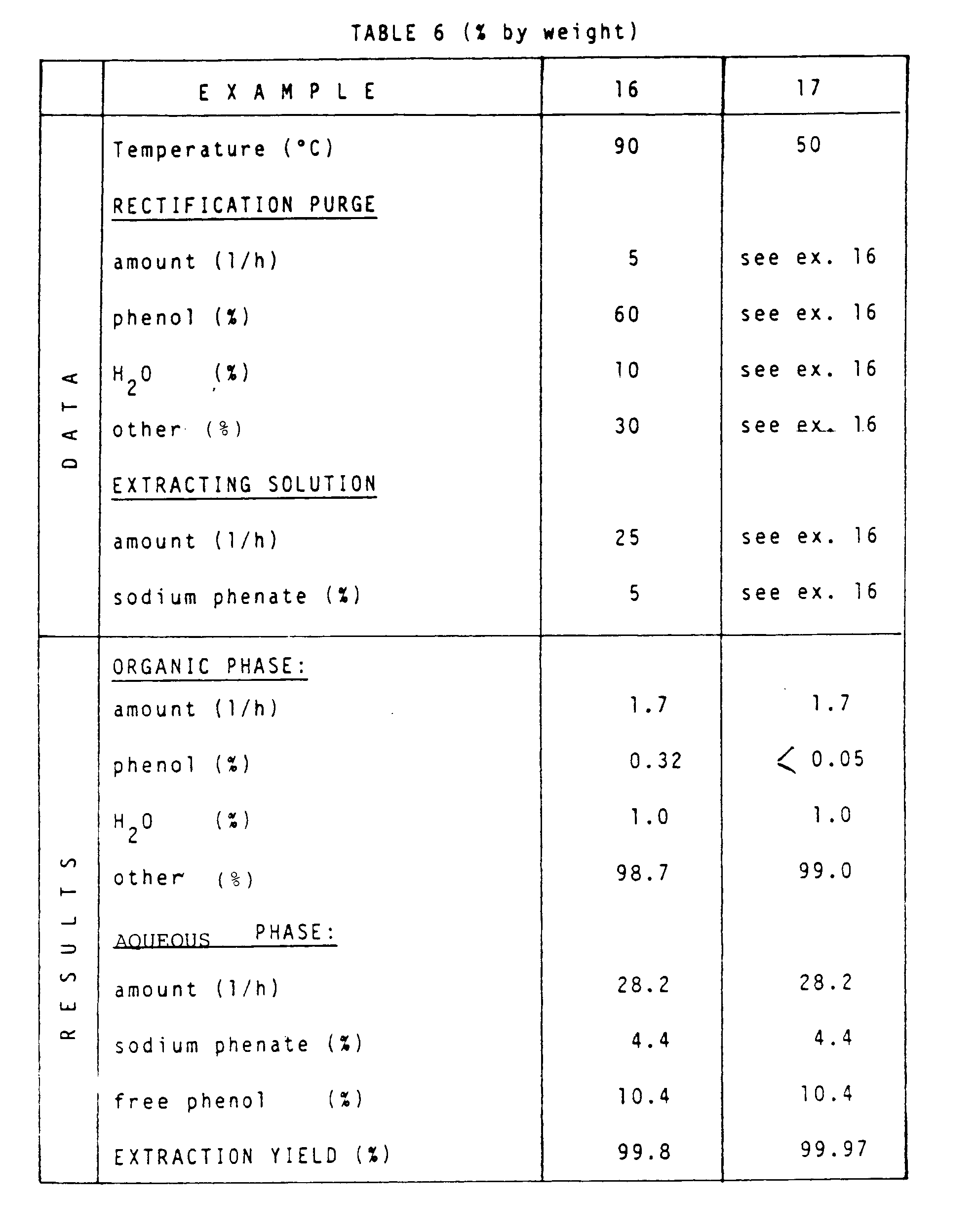

## Claims

1. A process for recovering phenol from a rectification purge, the purge comprising mesityl oxide, hydroxyacetone, cumene, α-methylstyrene and 2-methyl benzofuran and containing from 20 to 90% by weight of phenol, which process comprises subjecting the purge to an extraction operation, using, as an extracting solvent, an aqueous solution of an alkali metal phenate characterised in that the aqueous solution contains from 1 to 15% by weight of an alkali metal phenate which has been obtained in a process for the synthesis of phenol by cleavage of cumene hydroperoxide.

2. The process according to claim 1, wherein the phenate is sodium phenate.

3. The process according to claim 1 or claim 2, wherein the aqueous solution contains from 5 to 15% by weight, of phenate.

4. The process according to any one of the preceding claims, wherein the extraction temperature is in the range of from room temperature to 90°C.

5. The process according to claim 4 wherein the extraction temperature is in the range from 40 to 80°C.

6. The process according to any of the preceding claims, wherein the amount of extracting solvent is in the range of from 1 to 15 parts by weight per part of purge.

7. The process according to claim 6 wherein the amount of extracting solvent is in the range from 5 to 15 parts by weight per part of purge.

8. The process according to any of the preceding claims, wherein the extraction is carried out continuously, the flows of purge and extracting solvent being fed in countercurrent to an apparatus system consisting of two or more mixers and of two or more decanters.

9. The process according to any of the preceding claims, wherein the extraction is carried out continuously by flowing the purge and extracting solvent in countercurrent in an apparatus which allows a plurality of extraction steps, without requiring a physical separation of the two flows.

10. The process according to claim 9, wherein the organic phase is the continuous phase, while the aqueous phase is the dispersed phase.

11. The process according to claim 9 or claim 10, wherein the extraction is carried out in a tubular apparatus loaded with packing material.

12. The process according to claim 11, wherein the packing material is stainless steel.

13. The process according to claim 11 or claim 12 wherein the packing material is preferentially wet by the organic phase.

14. The process according to claim 12 or claim 13, wherein the packing material comprises Raschig rings or Pall rings.

## Patentansprüche

1. Verfahren zur Isolierung von Phenol aus einem Rektifikations-Reinigungsprodukt, das Mesityloxid, Hydroxyaceton, Cumol, α-Methylstyrol und 2-Methylbenzofuran umfaßt und 20 bis 90 Gew.-% Phenol enthält, umfassend das Extrahieren des Reinigungsprodukts, wobei man als extrahierendes Lösungsmittel eine wäßrige Lösung eines Alkalimetallphenats einsetzt, dadurch gekennzeichnet, daß die wäßrige Lösung 1 bis 15 Gew.-% eines Alkalimetallphenats enthält, das in einem Verfahren zur Synthese von Phenol durch Spaltung von Cumolhydroperoxid erhalten wurde.

2. Verfahren nach Anspruch 1, in welchem das Phenat Natrium-Phenat ist.

3. Verfahren nach Anspruch 1 oder Anspruch 2, in welchem die wäßrige Lösung 5 bis 15 Gew.-% Phenat enthält.

4. Verfahren nach irgendeinem der vorangehenden Ansprüche, in welchem die Extraktionstemperatur im Bereich von Raumtemperatur bis 90°C liegt.

5. Verfahren nach Anspruch 4, in welchem die Extraktionstemperatur im Bereich von 40 bis 80°C liegt.

6. Verfahren nach irgendeinem der vorangehenden Ansprüche, in welchem die Menge an extrahierendem Lösungsmittel im Bereich von 1 bis 15 Gew.-Teilen pro Teil Reinigungsprodukt liegt.

7. Verfahren nach Anspruch 6, in welchem die Menge an extrahierendem Lösungsmittel im Bereich von 5 bis 15 Gew.-Teilen pro Teil Reinigungsprodukt liegt.

8. Verfahren nach irgendeinem der vorangehenden Ansprüche, in welchem die Extraktion kontinuierlich durchgeführt wird, wobei die Ströme von Reinigungsprodukt und extrahierendem Lösungsmittel einem Vorrichtungssystem, das aus zwei oder mehr Mischern und aus zwei oder mehr Dekantern besteht, im Gegenstrom zugeführt werden.

9. Verfahren nach irgendeinem der vorangehenden Ansprüche, in welchem die Extraktion kontinuierlich durchgeführt wird, indem man das Reinigungsprodukt und das extrahierende Lösungsmittel in einer Apparatur, die eine Mehrzahl von Extraktionsstufen ermöglicht, ohne eine physikalische Trennung der zwei Ströme zu erfordern, im Gegenstrom zueinander führt.

10. Verfahren nach Anspruch 9, in welchem die organische Phase die kontinuierliche Phase ist, während die wäßrige Phase die dispergierte Phase ist.

11. Verfahren nach Anspruch 9 oder Anspruch 10, in welchem die Extraktion in einer mit Packungsmaterial beladenen röhrenförmigen Apparatur durchgeführt wird.

12. Verfahren nach Anspruch 11, in welchem das Packungsmaterial Edelstahl ist.

13. Verfahren nach Anspruch 11 oder Anspruch 12, in welchem das Packungsmaterial bevorzugt durch die organische Phase benetzt wird.

14. Verfahren nach Anspruch 12 oder 13, in welchem das Packungsmaterial Raschig-Ringe oder Pall-Ringe umfaßt.

## Revendications

1. Procédé de récupération de phénol à partir d'un liquide de purge de rectification, lequel liquide de purge contient de l'oxyde de mésityle, de l'hydroxyacétone, du cumène, de l'alpha-méthyl-styrène et du 2-méthyl-benzofuranne, ainsi que de 20 à 90 % en poids de phénol, lequel procédé comporte le fait de soumettre le liquide de purge à une opération d'extraction en utilisant, comme solvant d'extraction, une solution aqueuse d'un phénate de métal alcalin, caractérisé en ce que cette solution aqueuse contient de 1 à 15 % en poids d'un phénate de métal alcalin que l'on a obtenu au cours d'un procédé de synthèse de phénol par scission d'hydroperoxyde de cumène.

2. Procédé conforme à la revendication 1, dans lequel le phénate est du phénate de sodium.

3. Procédé conforme à la revendication 1 ou 2, dans lequel la solution aqueuse contient de 5 à 15 % en poids de phénate.

4. Procédé conforme à l'une des revendications précédentes, dans lequel la température d'extraction se situe dans l'intervalle allant de la température ambiante à 90°C.

5. Procédé conforme à la revendication 4, dans lequel la température d'extraction se situe dans l'intervalle allant de 40°C à 80°C.

6. Procédé conforme à l'une des revendications précédentes, dans lequel la quantité de solvant d'extraction se situe dans l'intervalle allant de 1 à 15 parties en poids par partie de liquide de purge.

7. Procédé conforme à la revendication 6, dans lequel la quantité de solvant d'extraction se situe dans l'intervalle allant de 5 à 15 parties en poids par partie de liquide de purge.

8. Procédé conforme à l'une des revendications précédentes, dans lequel on effectue l'extraction en mode continu, en envoyant les courants de liquide de purge et de solvant d'extraction à contre-courant dans une installation constituée d'au moins deux mélangeurs et d'au moins deux décanteurs.

9. Procédé conforme à l'une des revendications précédentes, dans lequel on effectue l'extraction en mode continu, en envoyant les courants de liquide de purge et de solvant d'extraction à contre-courant dans une installation qui permet de réaliser plusieurs étapes d'extraction sans qu'il soit nécessaire de séparer physiquement les deux courants.

10. Procédé conforme à la revendication 9, dans lequel la phase organique constitue la phase continue, tandis que la phase aqueuse constitue la phase dispersée.

11. Procédé conforme à la revendication 9 ou 10, dans lequel on effectue l'extraction dans un appareil tubulaire contenant un matériau de garnissage.

12. Procédé conforme à la revendication 11, dans lequel le matériau de garnissage est en acier inoxydable.

13. Procédé conforme à la revendication 11 ou 12, dans lequel le matériau de garnissage est mouillé de préférence par la phase organique.

14. Procédé conforme à la revendication 12 ou 13, dans lequel le matériau de garnissage est constitué d'anneaux de Raschig ou d'anneaux de Pall.
